# FASCICULE DE BREVET EUROPEEN

(11) **EP 2 068 718 B1**
(45) Date de publication et mention de la délivrance du brevet: **31.03.2010**
(21) Numéro de dépôt: 07820739.6
(22) Date de dépôt: 01.10.2007
(51) Int. Cl.: A61B 10/02

(54) **DISPOSITIF DE PRELEVEMENT DE TISSU BIOLOGIQUE POUR L'IDENTIFICATION DES ANIMAUX**
GERÄT ZUR ENTNAHME VON BIOLOGISCHEN GEWEBEPROBEN ZUR IDENTIFIZIERUNG VON TIEREN
DEVICE FOR SAMPLING BIOLOGICAL TISSUE FOR THE IDENTIFICATION OF ANIMALS

(30) Priorité: 06.10.2006 FR 0608800
(43) Date de publication de la demande: 17.06.2009
(73) Titulaire: Allflex Europe SAS, 35502 Vitre Cedex (FR)
(72) Inventeur: HILPERT, Jean-Jacques, F-35500 Vitre (FR)
(74) Mandataire: Ravina, Bernard
(86) Numéro de dépôt international: PCT/EP2007/060353
(87) Numéro de publication internationale: WO 2008/040692

(56) Documents cités:
- WO-A-2004/010773
- WO-A-2006/000869
- DE-U1- 29 824 186
- GB-A- 2 223 409

## Description

La présente invention appartient au domaine du marquage et de l'identification des animaux d'élevage.

Elle se rapporte à un dispositif de prélèvement de tissu biologique destiné à être utilisé dans une étiquette pour le marquage des animaux, la partie mâle de l'étiquette pour le marquage des animaux incluant le dispositif de prélèvement de tissu biologique. L'invention a également pour objet une étiquette auriculaire dont la partie mâle comprend un tel dispositif.

Ces dernières années, le marquage du bétail sur pied comme les vaches, les porcs ou les moutons est devenu obligatoire dans bon nombre de pays dans le monde. Ce marquage est généralement réalisé en appliquant une étiquette sur une ou sur les deux oreilles de l'animal, ladite étiquette portant des moyens d'identification qui permettent l'identification individuelle de chaque animal. Il existe de nombreuses versions d'étiquettes, toutes comprenant une partie femelle ou de réception et une partie mâle ou de perçage. Pour la pose d'une telle étiquette, l'oreille de l'animal est positionnée entre la partie mâle et la partie femelle et, à l'aide d'une pince, on relie les deux parties entre elles par poinçonnement à travers l'oreille. Une fois posée, l'étiquette est supposée être impossible à retirer, interdisant ainsi toute fraude. Dans ce but, il a été conçu un certain nombre de mécanismes de verrouillage qui permettent de retenir la partie mâle dans la partie femelle une fois que les deux pièces ont été assemblées sur l'oreille de l'animal.

Les brevets EP 0177201, EP 0913081, WO 95/25426 et EP 1037525 décrivent les mécanismes de verrouillage sous forme d'une bague, d'encoches et/ou d'un clip de fixation qui verrouillent la partie mâle dans la partie femelle après assemblage de l'étiquette.

De plus, il devient de plus en plus important, non seulement de pouvoir identifier un animal avec son étiquette correspondante, mais également de pouvoir prélever un échantillon de tissu biologique de l'animal ainsi marqué avec l'étiquette et, lors d'une étape ultérieure, d'être capable d'attribuer cet échantillon, à partir duquel un certain nombre de caractéristiques biologiques/biochimiques ont été déterminées, à l'animal en question.

Le brevet EP 1014861 décrit un dispositif pour prélever un échantillon biologique qui comprend une capsule de test avec un réceptacle pour l'échantillon et un couvercle pour la capsule. Le réceptacle de l'échantillon et le couvercle de la capsule forment une étiquette qui est appliquée dans le même temps que la capsule contenant l'échantillon est préparée et fermée. La capsule ainsi obtenue porte les mêmes informations d'étiquetage que l'étiquette qui a été appliquée mais elle peut en être séparée. L'échantillon contenu dans la capsule peut donc être amené à un laboratoire pour réaliser les tests qui sont nécessaires pour déterminer une maladie ou les capacités immunologiques de l'animal.

Le brevet EP 1088212 décrit un dispositif pour obtenir et préparer un échantillon de tissu biologique pour un diagnostic génétique moléculaire. Le dispositif possède un réceptacle pour recevoir l'échantillon et un moyen de prélèvement de l'échantillon, lequel pénètre dans le réceptacle après prélèvement de l'échantillon et le ferme de manière hermétique. Dans le réceptacle de réception de l'échantillon, est prévu un moyen de protection contre les enzymes qui dégradent l'ADN. Le moyen de collecte de l'échantillon traverse par pression l'oreille de l'animal et pénètre dans le réceptacle de récupération de l'échantillon qui est lui même associé à une étiquette comprenant une plaque à pointe et une plaque à trous. Le réceptacle de récupération de l'échantillon est relié de façon démontable avec la plaque à trous et peut en conséquence être retiré après prélèvement de l'échantillon alors que l'étiquette reste dans l'oreille de l'animal.

Le brevet EP 1372379 décrit une étiquette pour le marquage des animaux ayant un dispositif de prélèvement de substance organique. Dans cette étiquette, la partie mâle est configurée en deux éléments séparables. Le premier de ces deux éléments reste dans la partie femelle de l'étiquette une fois que l'étiquette a été appliquée, alors que l'autre élément est utilisé pour prélever un échantillon de substance organique et peut se déplacer d'avant en arrière dans le premier élément.

L'inconvénient des brevets EP 1014861 et EP 1088212 est que les échantillons ainsi produits sont stockés de manière sécurisée et hermétique dans les capsules respectives ainsi produites. Les analyses de laboratoire nécessitent l'ouverture des capsules ainsi fermées, qui est fastidieuse. Il faut en effet recourir à l'application d'une force importante ou à l'utilisation d'un outillage spécifique, ce qui rend toute la procédure pénible et longue.

L'inconvénient du dispositif du brevet EP 1372379 est que l'échantillon produit, bien qu'il soit transportable jusqu'au laboratoire d'analyse, est difficile à stocker et à conserver. En conséquence, les études en laboratoire doivent être réalisées dans un intervalle de temps de quelques heures à quelques jours après le prélèvement afin de s'assurer que l'échantillon ne s'est pas, au moins en partie, dégradé.

Le document GB 2 223 409 décrit un dispositif de prélèvement selon le préambule de la revendication 1.

Ainsi, l'objet de la présente invention est de fournir un dispositif de prélèvement de tissu biologique associé à une étiquette auriculaire qui autorise un accès facile à l'échantillon obtenu, et qui dans le même temps et malgré cet accès facile, permet de stocker l'échantillon sur de longues périodes de temps, c'est à dire de plusieurs mois à plusieurs années. Cette invention permet en outre d'utiliser l'outil de prélèvement avec une étiquette d'identification des animaux de type fermé, seul type d'identification aujourd'hui accepté dans la plupart des pays disposant de systèmes d'identification officiels.

Les inventeurs ont eu la surprise de découvrir qu'en fixant un réservoir rempli d'un agent conservateur à une extrémité d'une aiguille creuse, telle qu'une aiguille pour biopsie, il était possible de concevoir un système pouvant être utilisé pour l'obtention d'un échantillon biologique pendant la pose d'une étiquette d'oreille et que ce système permettait à la fois une accessibilité instantanée et aisée à l'échantillon et une longue durée de conservation de celui-ci. De manière pratique, une dose d'un produit conservateur destiné au traitement de l'échantillon, est enfermée dans un réservoir dont une des parois est conçue pour être rompue ou transpercée par l'extrémité de l'aiguille creuse qui lui est adjacente, en conséquence de quoi l'intérieur du réservoir est mis en relation avec la cavité de l'aiguille creuse. L'agent conservateur entre alors en contact avec l'échantillon prélevé, à l'extrémité tranchante de l'aiguille creuse. La région de la paroi du réservoir conçue pour être brisée ou transpercée est ici appelée cloison. Par une action de perçage de cette cloison, l'agent conservateur peut exercer son action de conservation sur l'échantillon se trouvant dans la cavité de l'aiguille creuse. L'extrémité de l'aiguille creuse contenant l'échantillon peut-être placée dans une coupelle ou sous un couvercle qui peut, en même temps, isoler l'extrémité de l'aiguille de manière réversible. Ce faisant, l'échantillon est préservé et, lors du retrait du couvercle, l'échantillon est toujours accessible dans la cavité de l'aiguille. On peut alors l'exposer à d'autres réactifs en plongeant simplement l'aiguille dans ces derniers ou en mettant l'échantillon dans un récipient de laboratoire approprié.

Dans un mode de réalisation préféré, le dispositif de prélèvement de tissu biologique est configuré de façon à pouvoir être monté sur une pipette de laboratoire, telle qu'une pipette Eppendorf, sous la forme d'un couvercle qui renferme l'extrémité distale de l'aiguille, c'est-à-dire la seconde extrémité, et l'échantillon qui se trouve à l'intérieur.

Les échantillons obtenus à l'aide du dispositif de prélèvement de tissu biologique selon la présente invention sont facilement analysables car ils ne sont pas enfermés dans une capsule difficile à ouvrir. Ils peuvent en outre être conservés pendant une période de 6 mois à 3 ans en permettant ainsi la détermination de paramètres biochimiques/biologiques des échantillons de tissu biologique et l'identification de l'animal correspondant même après une longue période de temps.

Plus précisément, les objectifs de la présente invention sont atteints grâce à un dispositif de prélèvement de tissu biologique comprenant :
- un réservoir apte à contenir un produit chimique pour traiter un échantillon de tissu biologique,
- une aiguille creuse possédant un cavité qui se termine par une première ouverture à une première extrémité et par une seconde ouverture à une seconde extrémité, ladite aiguille creuse ayant également une arête coupante à ladite seconde extrémité, apte à découper le tissu biologique dans le but de créer un échantillon qui est réceptionné dans ladite cavité lorsque l'on enfonce le dispositif de prélèvement dans le tissu biologique,
- une pièce de fixation de ladite aiguille creuse, formée d'un seul tenant avec le réservoir, ladite pièce de fixation possédant un canal qui part de la base du réservoir et se termine par un orifice, ledit canal ayant des dimensions aptes à maintenir la partie proximale de ladite aiguille creuse portant la première extrémité,
et en ce que ladite base dudit réservoir comprend une cloison qui est apte à être percée par ladite première extrémité de ladite aiguille creuse lorsque l'on enfonce le dispositif de prélèvement dans le tissu biologique.

Dans un mode de réalisation préféré, ladite cloison comprend une zone d'épaisseur réduite par rapport à l'épaisseur de la paroi adjacente qui entoure ladite cloison, ou adopte un relief conférant une moindre résistance à la pression, par exemple grâce à des perforations ou à une rainure ou à un endroit qui sera rompu par la première extrémité de l'aiguille creuse. Il connus de l'homme de l'art (le plasturgiste si le dispositif est réalisé en matière plastique). On recherchera un niveau de résistance à la pression assez bas pour que la cloison se rompe à l'aplomb de l'aiguille et que la paroi avoisinante du réservoir ne soit pas endommagée. On évitera toutefois un résistance trop faible qui conduirait la cloison à se rompre au moindre appui, et en particulier alors que l'aiguille creuse n'a pas encore pu pénétrer dans le tissus qu'elle doit couper pour prélever un échantillon.

Dans une version d'exécution préférée de l'invention, le canal de la pièce de fixation a un diamètre intérieur qui s'adapte à un diamètre extérieur de la partie proximale de l'aiguille creuse, de sorte que ladite partie proximale s'engage par friction dans ledit canal, et que, lorsque l'on enfonce le dispositif de prélèvement dans le tissu biologique, elle se déplace vers le réservoir en perçant la cloison à l'aide de sa première extrémité.

Dans une variante de réalisation, le réservoir comprenant la paroi, et la pièce de fixation, sont fabriqués en plastique, par exemple par moulage. La pièce de fixation et le réservoir peuvent être réalisés d'un seul tenant, de sorte que la pièce de fixation est intégrée au réservoir, en prolongement de la paroi. Ils peuvent également être fabriqués séparément, auquel cas dans le dispositif selon l'invention, la pièce de fixation est distincte dudit réservoir et est reliée à celui-ci par un moyen d'assemblage dans une deuxième étape. Dans un mode de réalisation alternatif, la pièce de fixation est surmoulée avec le réservoir sur l'aiguille creuse.

De préférence, dans le dispositif selon l'invention, la matière de l'aiguille creuse est choisie de telle sorte qu'elle n'altère pas l'échantillon biologique. Elle peut être faite en métal ou dans un matériau composite. De préférence on utilise une aiguille en métal.

Dans une variante de réalisation, ladite aiguille creuse possède un axe longitudinal s'étendant entre la première et la seconde extrémités, qui est perpendiculaire à la cloison transperçable du réservoir.

De préférence, l'aiguille creuse est maintenue perpendiculaire à ladite cloison du réservoir par l'intermédiaire du canal central de ladite pièce de fixation.

Dans une variante de réalisation avantageuse, ledit réservoir possède une ouverture permettant de le remplir avec un produit chimique.

Selon un mode de réalisation préféré, ledit réservoir contient un produit chimique pour traiter un échantillon de tissu biologique, de préférence un agent conservateur apte à conserver un échantillon de tissu biologique.

De préférence, l'agent conservateur est sélectionné dans le groupe comprenant les agents de séchage, les inhibiteurs d'enzyme et les substances susceptibles d'empêcher le développement d'organismes bactériens. Un agent de séchage peut être par exemple le gel de silice, le sulfate de calcium, l'oxyde de magnésium, les pyrophosphates. Parmi les inhibiteurs d'enzyme on préfère les inhibiteurs des enzymes dégradant l'ADN. De manière préférée on place dans le réservoir une substance ou un mélange de substances ayant une action limitant le développement d'organismes bactériens.

Selon une caractéristique intéressante du dispositif selon l'invention, l'ouverture du réservoir est fermée par une feuille métallique scellée. Le dispositif selon l'invention se présente donc de préférence, dans sa forme prête à l'usage, avec son réservoir préalablement rempli du produit chimique choisi et scellé par un opercule. Par exemple, ledit réservoir qui contient un agent conservateur, a son ouverture scellée par une feuille aluminium.

Selon une caractéristique intéressante de la présente invention, le réservoir comprend sur sa partie externe un collier qui permet l'utilisation du dispositif de prélèvement sur la partie mâle d'une étiquette auriculaire de marquage des animaux, ledit collier ayant des dimensions qui lui permettent de s'engager dans l'une des mâchoires d'une pince utilisée pour la pose de telles étiquettes.

Dans un mode de réalisation du dispositif de prélèvement de tissu biologique selon l'invention, ledit dispositif possède un premier moyen d'identification.

Dans une variante préféré de la présente invention, dans le dispositif de prélèvement de tissu biologique, la paroi du réservoir comporte des moyens d'association avec un couvercle en forme de capuchon. Par exemple, le réservoir peut posséder à sa base une partie qui est dimensionnée pour s'engager dans un couvercle en forme de capuchon. Dans une version améliorée, ladite partie basale est une partie extérieure dudit réservoir. Ainsi, le dispositif de prélèvement de tissu biologique selon l'invention est de préférence doté de moyens d'association placés sur la face externe à la base du réservoir.

De manière avantageuse, la base du réservoir portant les moyens d'association avec le couvercle en forme de capuchon est prolongée d'un seul tenant par la pièce de fixation de l'aiguille creuse.

Dans un mode de réalisation particulier du dispositif de prélèvement de tissu biologique selon l'invention, les moyens d'association du réservoir avec un couvercle en forme de capuchon possèdent une collerette apte à maintenir ledit couvercle en forme de capuchon, de façon à ce que celui-ci recouvre la partie distale de l'aiguille creuse contenant éventuellement un échantillon de tissu biologique.

Autrement dit, la base du réservoir possède une collerette qui s'engage dans le couvercle en forme de capuchon, lorsque ledit couvercle est monté sur ladite base, de façon à ce que la seconde extrémité de l'aiguille creuse, et éventuellement l'échantillon de tissu biologique se trouvant dans la cavité de l'aiguille, soit protégée par le couvercle en forme de capuchon.

De manière avantageuse, le couvercle en forme de capuchon est fourni avec les autres éléments du dispositif de prélèvement. C'est pourquoi est également objet de la présente invention un dispositif de prélèvement de tissu biologique tel que décrit précédemment, qui comprend en outre un couvercle en forme de capuchon monté sur la collerette dudit dispositif de prélèvement de tissu biologique.

Selon un mode de réalisation particulier du dispositif de prélèvement de tissu biologique selon l'invention, le couvercle en forme de capuchon comporte un support du premier moyen d'identification, ledit premier moyen d'identification se prolongeant à partir dudit dispositif de prélèvement.

Dans un mode de réalisation préféré du dispositif selon l'invention, le couvercle en forme de capuchon est réalisé dans une matière transparente qui permet d'observer le prélèvement biologique.

Un autre objet de la présente invention est une étiquette auriculaire pour le marquage des animaux comportant :
i) une partie femelle, et
ii) une partie mâle comprenant une tige munie d'une pointe distale possédant un épaulement permettant le verrouillage de ladite partie mâle dans ladite partie femelle, ladite tige étant percée d'un canal axial dans toute sa longueur, et ladite partie mâle comprenant un dispositif de prélèvement de tissu biologique tel que décrit précédemment.

Dans un mode de réalisation avantageux de l'étiquette auriculaire selon l'invention, l'aiguille creuse dudit dispositif de prélèvement de tissu biologique est logée dans le canal axial de la tige, celle-ci ayant une longueur telle que l'arête coupante de la seconde extrémité de ladite aiguille creuse dépasse de la pointe de ladite tige, de manière à découper un échantillon de tissu biologique lorsque la partie mâle est enfoncée à travers l'oreille d'un animal.

Dans l'étiquette auriculaire selon le mode de réalisation ci-dessus, la seconde extrémité de l'aiguille creuse dépasse de la pointe de la tige d'une longueur au moins égale à l'épaisseur de la cloison. Ainsi, après avoir pénétré dans le tissu et en avoir prélevé une fraction, l'aiguille coulisse dans la tige mâle jusqu'à être totalement logée dans ladite tige, la translation devant amener la première extrémité de l'aiguille à travers la cloison mince jusque dans le réservoir. Cette translation doit donc être suffisante pour décaler l'aiguille jusqu'à l'intérieur du réservoir, sans le traverser entièrement bien évidemment.

Selon un caractéristique intéressante, l'étiquette auriculaire selon l'invention comprend en outre au moins un des éléments suivants :
- un second moyen d'identification porté par une plaque associée à la tige de la partie mâle,
- un troisième moyen d'identification porté par la partie femelle,
en conséquence de quoi au moins un du second ou du troisième moyen d'identification se trouve présent dans ladite étiquette auriculaire.

Il est bien entendu que la partie mâle comprenant le dispositif de prélèvement tel que décrit précédemment fait l'objet de la présente invention en tant qu'élément constitutif de l'étiquette auriculaire et en tant que tel.

L'invention sera mieux comprise à la lumière de l'exemple de réalisation ci-après, illustré par les figures dans lesquelles :
la figure 1 est un vue en coupe longitudinale d'un dispositif de prélèvement de tissu biologique selon la présente invention,
la figure 2 est une vue en coupe du même dispositif de prélèvement de tissu biologique, inséré dans la partie mâle d'une étiquette auriculaire,
la figure 3 : vue en coupe même dispositif, après prélèvement de tissu biologique et association avec un couvercle de protection en forme de capuchon.

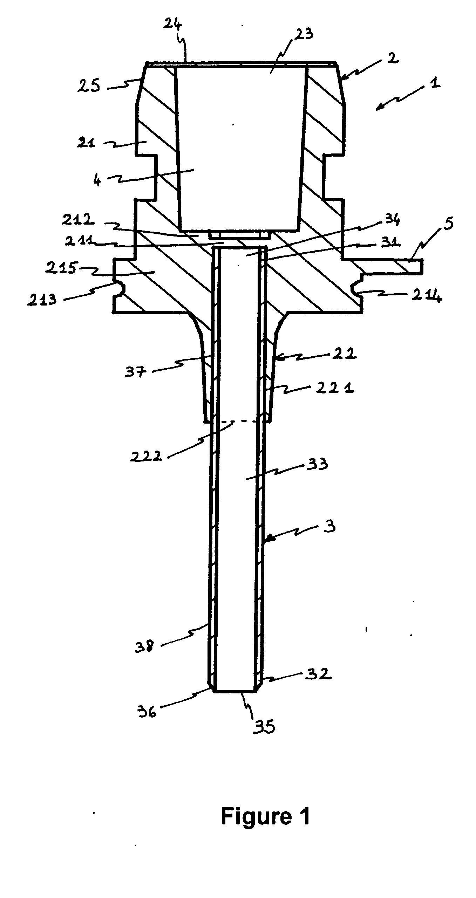

Le dispositif de prélèvement de tissu biologique 1, tel que représenté à la figure 1, comprend le réservoir 2 qui contient un agent conservateur 4 pour conserver un tissu biologique. Le réservoir 2 possède la paroi 21 comprenant la paroi latérale et la base 215. Il est doté de l'ouverture 23 fermée par la feuille 24. L'ouverture 23 de remplissage du réservoir 2 est ici située à l'opposé de la base 215. Après avoir terminé l'opération de remplissage, le réservoir est fermé par scellement de la feuille 24. De préférence, cette feuille sera en plastique ou en métal ou une association des deux.

L'aiguille creuse 3, dont la première extrémité 21 est adjacente au réservoir 2, et séparé de lui par la cloison mince 211, est maintenue par la pièce de fixation 22 de forme conique. Cette pièce de fixation 22 possède le canal intérieur 221 qui s'étend de la cloison 211 du réservoir 2, jusqu'à l'orifice 35 de l'extrémité opposée 32 (seconde extrémité). L'aiguille 3 peut se déplacer le long de son axe longitudinal dans le canal 221, et si on exerce une pression à la seconde extrémité 32 de l'aiguille 3, qui est distale par rapport au réservoir 2, l'aiguille 3 se déplace vers le réservoir 2. Si la pression ainsi exercée est assez importante, l'aiguille 3 traverse la cloison 211 du réservoir 2 avec sa première extrémité 31, perçant ainsi le réservoir 2 et mettant en relation le volume intérieur de l'aiguille creuse 3 et du réservoir 2.

Avant la prise d'échantillon, l'aiguille creuse 3 est maintenue en position perpendiculaire au réservoir 2 et sa première extrémité 31 à proximité du réservoir 2, par l'intermédiaire de la pièce de fixation 22 qui fait ici partie intégrante du réservoir 2. Le réservoir incluant la paroi latérale et la base 215 ainsi que la pièce de fixation 22 sont en matière plastique.

Le canal 33 de la pièce de fixation 22 est dimensionné de façon à ce qu'il reçoive l'aiguille creuse 3 par friction. Ceci veut dire que pour déplacer l'aiguille creuse 3 dans le canal 22, il faut exercer une certaine pression, telle que la pression qui est exercée lorsque la partie mâle d'une étiquette auriculaire est enfoncée dans l'oreille d'un animal. Cette action est suffisante pour pousser l'aiguille 3 en retrait sur une courte distance dans le canal 221 et pour de percer la cloison 211 du réservoir 2 contenant l'agent conservateur 4, et en même temps, enfoncer l'arrête coupante 36 de l'extrémité 32 de l'aiguille 3 dans le tissu auriculaire de l'animal de sorte qu'un petit cylindre de tissu est découpé et vient se loger par l'ouverture 35 à l'intérieur de l'aiguille creuse 3.

L'aiguille creuse 3 peut être en métal, en plastique dur, ou en tout autre matière connue de l'homme de l'art, dès lors que ce matériau est capable de supporter les pressions que l'on exerce usuellement lors de la pose d'une étiquette auriculaire à un animal et/ou pendant le prélèvement d'un échantillon.

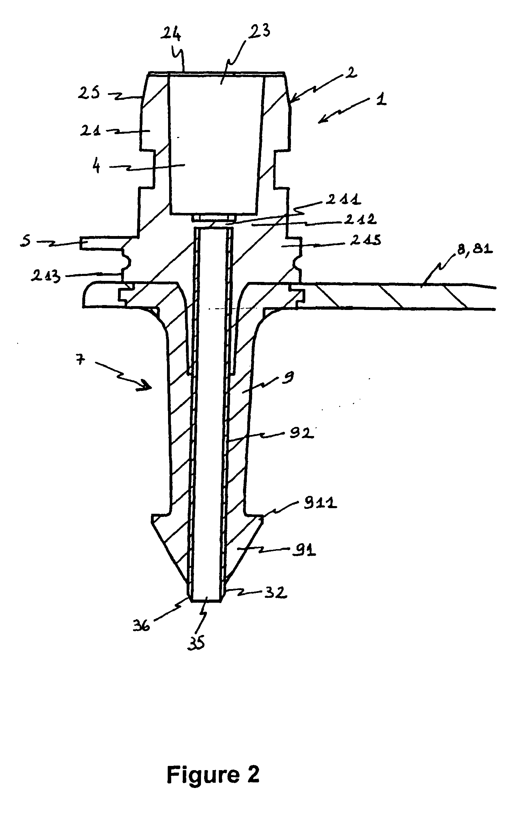

Le dispositif de prélèvement 1 de tissu biologique est avantageusement utilisé en association avec la partie mâle 7 d'une étiquette auriculaire 1, comme représenté sur la figure 2. La partie mâle 7 possède la plaque 8, la tige 9 solidaire de la plaque 8, se terminant par la pointe distale 91 qui possède un épaulement 911 ce qui permet le verrouillage de la partie mâle 7 à l'intérieur d'une partie femelle de l'étiquette (non représentée). Dans la tige 9 se trouve le canal axial 92 qui s'étend de la plaque 8 jusqu'à la pointe distale 91, et qui sert à recevoir l'aiguille 3 du dispositif de prélèvement 1 de tissu biologique. La longueur de la tige 9 a été étudiée pour que la seconde extrémité 32 de l'aiguille creuse 3, une fois celle-ci insérée entièrement dans la tige 9, dépasse de la tige 9, c'est-à-dire que l'arête coupante 36 de l'extrémité 32 est libre, et peut trancher le tissu en permettant ainsi la prise d'un échantillon de tissu biologique lorsque la pointe 91 de la partie mâle 7, incluant le dispositif de prélèvement 1 de tissu biologique, est enfoncé à travers l'oreille de l'animal dans la partie femelle de l'étiquette. Le dispositif de prélèvement 1 de tissu biologique selon la présente invention possède également le moyen d'identification 5 qui porte l'information relative à l'identité de l'animal ainsi marqué et échantillonné. Le moyen d'identification 5 du dispositif de prélèvement 1 de tissu biologique selon la présente invention peut être par exemple, un moyen visuel ou un transpondeur avec un numéro électronique. Si la partie mâle 7 ou la partie femelle, possède aussi un moyen d'identification, ces deuxième et troisième moyens 81, 111, peuvent être visuels ou être des transpondeurs portant des informations.

La figure 2 montre que la base 215 du réservoir 2 est en contact avec la plaque 8 de la partie mâle 7 de l'étiquette, lorsque l'aiguille creuse 3 est entièrement insérée dans la tige 9 de ladite partie mâle. Le réservoir 2 du dispositif de prélèvement 1 possède de plus sur sa partie extérieure le collier 25 qui permet le montage d'un ensemble constitué du dispositif de prélèvement 1 et de la partie mâle 7 sur la mâchoire d'une pince de pose standard.

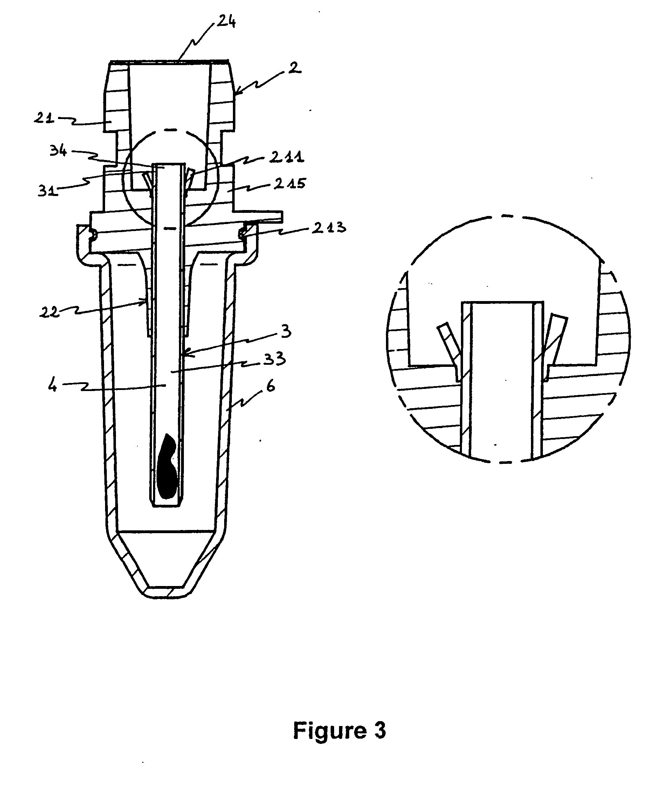

De plus, la base 215 du réservoir 2 possède la collerette 214 qui peut s'engager dans le couvercle 6 en forme de capuchon tel que représenté à la figure 3. Dans certains modes de réalisation, le collier 25 ci-dessus décrit peut faire partie de la collerette 214 ou peut être la même pièce que ladite collerette. Dans d'autres variantes de réalisation, le collier 25 et la collerette 214 sont distincts. Les dimensions de la collerette 214 sont calculées de sorte qu'elle puisse s'engager dans un couvercle 6 en forme de capuchon qui, dans une variante préférée, est un article standard de laboratoire, comme une pipette Eppendorf. Lorsqu'un couvercle de ce type est monté sur la base 215 du réservoir 2, l'extrémité 32 de l'aiguille 3 contenant l'échantillon se retrouve dans ledit couvercle en forme de capuchon et peut être ensuite soumise à des réactions chimiques ou être placée dans une centrifugeuse de laboratoire, comme par exemple une centrifugeuse portative pour pipettes Eppendorf.

Un dispositif de prélèvement 1 de tissu biologique muni d'un couvercle 6 en forme de capuchon tel qu'il se présente après obtention d'un échantillon est montré à la figure 3. Le moyen d'identification 5 attaché au dispositif de prélèvement 1 de tissu biologique permet l'identification de l'échantillon dans l'aiguille creuse 3. La vue en coupe de la figure 3 montre que l'aiguille 3 a été poussée dans le réservoir 2 en perçant et en traversant la cloison 211.

## Revendications

1. Dispositif de prélèvement de tissu biologique (1) comprenant:
- un réservoir (2) apte à contenir un produit chimique pour traiter un échantillon de tissu biologique,
- une aiguille creuse (3) possédant un cavité (33) qui se termine par une première ouverture (34) à une première extrémité (31) et par une seconde ouverture (35) à une seconde extrémité (32), ladite aiguille creuse ayant également une arête coupante (36) à ladite seconde extrémité, apte à découper le tissu biologique dans le but de créer un échantillon qui est réceptionné dans ladite cavité lorsque l'on enfonce le dispositif de prélèvement dans le tissu biologique,
- une pièce de fixation (22) de ladite aiguille creuse, ladite pièce de fixation possédant un canal (221) qui part de la base (214) du réservoir (2) et se termine par un orifice (222), ledit canal ayant des dimensions aptes à maintenir la partie proximale (37) de ladite aiguille creuse portant la première extrémité (31),
et en ce que ladite base dudit réservoir comprend une cloison (211) qui est apte à être percée par ladite première extrémité de ladite aiguille creuse lorsque l'on enfonce le dispositif de prélèvement dans le tissu biologique, **caractérisé en ce que** la pièce de fixation est formée d'un seul tenant avec le réservoir (2).

2. Dispositif de prélèvement de tissu biologique selon la revendication 1 **caractérisé en ce que** la cloison (211) comprend une zone d'épaisseur réduite par rapport à l'épaisseur de la paroi adjacente (212) qui entoure ladite cloison, ou adopte un relief conférant une moindre résistance à la pression.

3. Dispositif de prélèvement de tissu biologique selon la revendication 1 ou 2, **caractérisé en ce que** le canal (221) a un diamètre intérieur qui s'adapte à un diamètre extérieur de la partie proximale (37) de l'aiguille creuse (3), de sorte que ladite partie proximale s'engage par friction dans ledit canal, et que, lorsqu'on enfonce le dispositif de prélèvement (1) dans le tissu biologique, elle se déplace vers le réservoir (2) en perçant la cloison (211) à l'aide de la première extrémité (31).

4. Dispositif de prélèvement de tissu biologique selon l'une quelconque des revendications précédentes **caractérisé en ce que** le réservoir (2) comprenant la paroi (21), et la pièce de fixation (22) sont fabriqués en plastique.

5. Dispositif de prélèvement de tissu biologique selon l'une quelconque des revendications précédentes **caractérisé en ce que** la matière de l'aiguille creuse (3) n'altère pas l'échantillon biologique.

6. Dispositif de prélèvement de tissu biologique selon l'une quelconque des revendications précédentes **caractérisé en ce que** l'aiguille creuse (3) possède un axe longitudinal s'étendant entre la première et la seconde extrémités (31, 32) qui est perpendiculaire à la cloison (211) du réservoir (2).

7. Dispositif de prélèvement de tissu biologique selon l'une quelconque des revendications précédentes **caractérisé en ce que** l'aiguille creuse (3) est maintenue perpendiculaire à la cloison (211) du réservoir (2) par l'intermédiaire du canal (221) de la pièce de fixation (22).

8. Dispositif de prélèvement de tissu biologique selon l'une quelconque des revendications précédentes **caractérisé en ce que** le réservoir (2) possède une ouverture (23) permettant de le remplir avec un produit chimique.

9. Dispositif de prélèvement de tissu biologique selon l'une quelconque des revendications précédentes **caractérisé en ce que** le réservoir (2) contient un produit chimique de traitement d'un échantillon de tissu biologique, de préférence un agent conservateur apte à conserver un échantillon de tissu biologique.

10. Dispositif de prélèvement de tissu biologique selon la revendication 9 **caractérisé en ce que** l'agent conservateur est sélectionné dans le groupe comprenant les agents de séchage, les inhibiteurs d'enzyme et les substances susceptibles d'empêcher le développement d'organismes bactériens.

11. Dispositif de prélèvement de tissu biologique selon l'une quelconque des revendications 9 ou 10 **caractérisé en ce que** l'ouverture (23) du réservoir (2) est fermée par une feuille métallique scellée (24).

12. Dispositif de prélèvement de tissu biologique selon l'une quelconque des revendications précédentes **caractérisé en ce que** le réservoir (2) comprend sur sa partie externe un collier (25) qui permet l'utilisation dudit dispositif de prélèvement sur la partie mâle d'une étiquette auriculaire de marquage des animaux, ledit collier ayant des dimensions qui lui permettent de s'engager dans l'une des mâchoires d'une pince utilisée pour la pose de telles étiquettes.

13. Dispositif de prélèvement de tissu biologique selon l'une quelconque des revendications précédentes **caractérisé en ce que** ledit dispositif de prélèvement de tissu biologique possède un premier moyen d'identification (5).

14. Dispositif de prélèvement de tissu biologique selon l'une quelconque des revendications précédentes **caractérisé en ce que** la paroi (21) du réservoir (2) comporte des moyens (213) d'association avec un couvercle (6) en forme de capuchon.

15. Dispositif de prélèvement de tissu biologique selon la revendication 14 **caractérisé en ce que** les moyens d'association (213) sont placés sur la face externe à la base (215) du réservoir (2).

16. Dispositif de prélèvement de tissu biologique selon l'une quelconque des revendications 14 ou 15 **caractérisé en ce que** la base (215) du réservoir (2) portant les moyens d'association (213) avec le couvercle (6) en forme de capuchon est prolongée d'un seul tenant par la pièce de fixation (22) de l'aiguille creuse (3).

17. Dispositif de prélèvement de tissu biologique selon l'une quelconque des revendications 14 à 16 **caractérisé en ce que** les moyens d'association (213) possèdent une collerette (214) apte à maintenir ledit couvercle en forme de capuchon, de façon à ce que celui-ci recouvre la partie distale (38) de l'aiguille creuse (3) contenant éventuellement un échantillon de tissu biologique.

18. Dispositif de prélèvement de tissu biologique selon l'une quelconque des revendications 14 à 17 **caractérisé en ce qu'**il est comprend en outre un couvercle (6) en forme de capuchon monté sur la collerette (214) dudit dispositif de prélèvement de tissu biologique.

19. Dispositif de prélèvement de tissu biologique selon la revendication 18 **caractérisé en ce que** le couvercle (6) en forme de capuchon comporte un support (51) du premier moyen d'identification (5), ledit premier moyen d'identification se prolongeant à partir dudit dispositif de prélèvement.

20. Dispositif de prélèvement de tissu biologique selon les revendications 18 ou 19 **caractérisé en ce que** le couvercle (6) en forme de capuchon est réalisé dans une matière transparente qui permet d'observer le prélèvement biologique.

21. Etiquette auriculaire pour le marquage des animaux comportant i) une partie femelle et ii) une partie mâle (7) comprenant une tige (9) munie d'une pointe distale (91) possédant un épaulement (911) permettant le verrouillage de ladite partie mâle dans ladite partie femelle, ladite tige étant percée d'un canal axial (92) dans toute sa longueur, et ladite partie mâle comprenant un dispositif de prélèvement de tissu biologique (1) selon l'une quelconque des revendications 1 à 20.

22. Etiquette auriculaire selon la revendication précédente **caractérisée en ce que** l'aiguille creuse (3) dudit dispositif de prélèvement de tissu biologique est logée dans le canal axial (92) de la tige (9), celle-ci ayant une longueur telle que l'arête coupante (36) de la seconde extrémité (32) de ladite aiguille creuse dépasse de la pointe (91) de ladite tige, de manière à découper un échantillon de tissu biologique lorsque la partie mâle (7) est enfoncée à travers l'oreille d'un animal.

23. Etiquette auriculaire selon l'une des revendications 21 ou 22 **caractérisée en ce** la seconde extrémité (32) de l'aiguille creuse (3) dépasse de la pointe (91) de la tige (22) d'une longueur au moins égale à l'épaisseur de la cloison (211).

24. Etiquette auriculaire selon l'une des revendications 21 à 23 **caractérisée en ce qu'**elle comprend en outre au moins un des éléments suivants :
- un second moyen d'identification (81) porté par une plaque (8) associée à la tige (9),
- un troisième moyen d'identification (111) porté par la partie femelle,
en conséquence de quoi au moins un du second ou du troisième moyen d'identification se trouve présent dans ladite étiquette auriculaire.

## Claims

1. Device (1) for sampling biological tissue, comprising:
- a tank (2) that can contain a chemical product for treating a sample of biological tissue,
- A hollow needle (3) that has a cavity (33) that ends by a first opening (34) at a first tip (31) and by a second opening (35) at a second tip (32), said hollow needle also having a cutting edge (36) at said second tip that is able to cut out the biological tissue for the purpose of creating a sample that is received in said cavity when the sampling device is pushed into the biological tissue,
- an attachment part (22) of said hollow needle, said attachment part having a channel (221) that emerges from the base (214) of the tank (2) and ends in an orifice (222), said channel having dimensions that can hold the proximal part (37) of said hollow needle that carries the first tip (31),
and said tank comprising a partition (211) that can be pierced by said first tip of said hollow needle when the sampling device is pushed into the biological tissue, **characterized in that** the attachment part is formed integral with the tank (2).

2. Device for sampling biological tissue according to claim 1, **characterized in that** the partition (211) comprises a zone of reduced thickness relative to the thickness of the adjacent wall (212) that surrounds said partition or adopts a relief that imparts less resistance to the pressure.

3. Device for sampling biological tissue according to claim 1 or 2, **characterized in that** the channel (221) has an inside diameter that is adapted to an outside diameter of the proximal part (37) of the hollow needle (3), such that said proximal part is engaged by friction in said channel and such that when the sampling device (I) is pushed into the biological tissue, it moves toward the tank (2) by piercing the partition (211) using the first tip (31).

4. Device for sampling biological tissue according to any of the preceding claims, **characterized in that** the tank (2) that comprises the wall (21) and the attachment part (22) are made of plastic.

5. Device for sampling biological tissue according to any of the preceding claims, **characterized in that** the material of the hollow needle (3) does not alter the biological sample.

6. Device for sampling biological tissue according to any of the preceding claims, **characterized in that** the hollow needle (3) has a longitudinal shaft that extends between the first and second tips (31, 32) and that is perpendicular to the partition (211) of the tank (2).

7. Device for sampling biological tissue according to any of the preceding claims, **characterized in that** the hollow needle (3) is kept perpendicular to the partition (211) of the tank (2) by means of the channel (221) of the attachment part (22).

8. Device for sampling biological tissue according to any of the preceding claims, **characterized in that** the tank (2) has an opening (23) that makes it possible to fill it with a chemical product.

9. Device for sampling biological tissue according to any of the preceding claims, **characterized in that** the tank (2) contains a chemical product for treatment of a sample of biological tissue, preferably a preservative that can preserve a sample of biological tissue.

10. Device for sampling biological tissue according to claim 9, **characterized in that** the preservative is selected from the group that comprises drying agents, enzyme inhibitors, and substances that can prevent the development of bacterial organisms.

11. Device for sampling biological tissue according to any of claims 9 or 10, **characterized in that** the opening (23) of the tank (2) is closed by a sealed metal sheet (24).

12. Device for sampling biological tissue according to any of the preceding claims, **characterized in that** on its outside part, the tank (2) comprises a collar (25) that allows the use of said sampling device on the male part of an ear tag for tagging animals, said collar having dimensions that make it possible for it to engage in one of the jaws of a pair of pliers used for placing such tags.

13. Device for sampling biological tissue according to any of the preceding claims, **characterized in that** said device for sampling biological tissue has a first identification means (5).

14. Device for sampling biological tissue according to any of the preceding claims, **characterized in that** the wall (21) of the tank (2) comprises means (213) of association with a cap-shaped cover (6).

15. Device for sampling biological tissue according to claim 14, **characterized in that** the means of association (213) are placed on the outside face at the base (215) of the tank (2).

16. Device for sampling biological tissue according to any of claims 14 or 15, **characterized in that** the base (215) of the tank (2) that carries the means of association (213) with the cap-shaped cover (6) is extended integral with the attachment part (22) of the hollow needle (3).

17. Device for sampling biological tissue according to any of claims 14 to 16, **characterized in that** the means of association (213) have a flange (214) that can hold said cap-shaped cover so that the latter covers the distal part (38) of the hollow needle (3) that optionally contains a biological tissue sample.

18. Device for sampling biological tissue according to any of claims 14 to 17, **characterized in that** it also comprises a cap-shaped cover (6) that is mounted on the flange (214) of said device for sampling biological tissue.

19. Device for sampling biological tissue according to claim 18, **characterized in that** the cap-shaped cover (6) comprises a support (51) of the first identification means (5), said first identification means extending from said sampling device.

20. Device for sampling biological tissue according to claim 18 or 19, **characterized in that** the cap-shaped cover (6) is made of a transparent material that makes it possible to observe the biological sampling.

21. Ear tag for tagging animals that comprises (i) a female part, and ii) a male part (7) comprising a rod (9) that is equipped with a distal point (91) that has a shoulder (911) allowing the locking of said male part in said female part, said rod being pierced by an axial channel (92) in its entire length, and said male part comprising a device for sampling biological tissue (1) according to any of claims 1 to 20.

22. Ear tag according to the preceding claim, **characterized in that** the hollow needle (3) of said device for sampling biological tissue is housed in the axial channel (92) of the rod (9), the latter having a length such that the cutting edge (36) of the second tip (32) of said hollow needle goes beyond the point (91) of said rod so as to cut out a sample of biological tissue when the male part (7) is pushed through the ear of an animal.

23. Ear tag according to one of claims 21 or 22, **characterized in that** the second tip (32) of the hollow needle (3) goes beyond the point (91) of the rod (22) by a length that is at least equal to the thickness of the partition (211).

24. Ear tag according to one of claims 21 to 23, **characterized in that** it also comprises at least one of the following elements:
- a second means of identification (81) that is carried by a plate (8) that is combined with the rod (9),
- a third means of identification (111) that is carried by the female part,
ensuring that at least one of the second or third means of identification is found in said ear tag.

## Patentansprüche

1. Vorrichtung zur Entnahme biologischen Gewebes (1) umfassend:
- einen Behälter (2), der dazu geeignet ist, einen chemischen Stoff zur Behandlung einer Probe biologischen Gewebes zu enthalten,
- eine Hohlnadel (3), die einen Hohlraum (33) aufweist, welcher an einem ersten Ende (31) in einer ersten Öffnung (34) endet und an einem zweiten Ende (32) in einer zweiten Öffnung (35) endet, wobei die Hohlnadel am zweiten Ende weiterhin eine Schnittkante (36) hat, die dazu geeignet ist, das biologische Gewebe abzuschneiden, um eine Probe zu erhalten, welche in dem Hohlraum aufgenommen wird, wenn die Vorrichtung zur Entnahme in das biologische Gewebe gedrückt wird,
- ein Teil zur Befestigung (22) der Hohlnadel, wobei das Teil zur Befestigung einen Kanal (221) aufweist, der von der Basis (214) des Behälters (2) ausgeht und in einer Öffnung (222) endet, wobei der Kanal Abmessungen hat, die dazu geeignet sind, den proximalen Abschnitt (37) der Hohlnadel, an welchem sich das erste Ende (31) befindet, festzuhalten,
und **dadurch**, dass die Basis des Behälters eine Trennwand (211) umfasst, die dazu geeignet ist, vom ersten Ende der Hohlnadel durchstochen zu werden, wenn die Vorrichtung zur Entnahme in das biologische Gewebe gedrückt wird, **dadurch gekennzeichnet, dass** das Teil zur Befestigung einstückig mit dem Behälter (2) gebildet ist.

2. Vorrichtung zur Entnahme biologischen Gewebes nach Anspruch 1, **dadurch gekennzeichnet, dass** die Trennwand (211) einen Bereich umfasst, dessen Dicke gegenüber der Dicke der angrenzenden Wand (212), welche die Trennwand umgibt, verringert ist, oder eine Oberflächenbeschaffenheit aufweist, die einen geringeren Druckwiderstand verleiht.

3. Vorrichtung zur Entnahme biologischen Gewebes nach Anspruch 1 oder 2, **dadurch gekennzeichnet, dass** der Kanal (221) einen Innendurchmesser hat, der sich dem Außendurchmesser des proximalen Abschnitts (37) der Hohlnadel (3) anpasst, sodass der proximale Abschnitt durch Reibebewegungen in den Kanal eingeführt werden kann und dass er sich, wenn die Vorrichtung zur Entnahme (1) in das biologische Gewebe gedrückt wird, in Richtung des Behälters (2) bewegt, wobei er mit Hilfe des ersten Endes (31) die Trennwand (211) durchsticht.

4. Vorrichtung zur Entnahme biologischen Gewebes nach einem beliebigen der vorhergehenden Ansprüche **dadurch gekennzeichnet, dass** der Behälter (2), der die Wand (21) umfasst, sowie das Teil zur Befestigung (22) aus Kunststoff hergestellt sind.

5. Vorrichtung zur Entnahme biologischen Gewebes nach einem beliebigen der vorhergehenden Ansprüche, **dadurch gekennzeichnet, dass** das Material der Hohlnadel (3) die biologische Probe nicht beeinträchtigt.

6. Vorrichtung zur Entnahme biologischen Gewebes nach einem beliebigen der vorhergehenden Ansprüche, **dadurch gekennzeichnet, dass** die Hohlnadel (3) eine Längsachse aufweist, die sich zwischen dem ersten und dem zweiten Ende (31, 32) erstreckt und rechtwinklig zur Trennwand (211) des Behälters (2) ist.

7. Vorrichtung zur Entnahme biologischen Gewebes nach einem beliebigen der vorhergehenden Ansprüche, **dadurch gekennzeichnet, dass** die Hohlnadel (3) durch den Kanal (221) des Teils zur Befestigung (22) in rechtwinkliger Stellung zur Trennwand (211) des Behälters (2) gehalten wird.

8. Vorrichtung zur Entnahme biologischen Gewebes nach einem beliebigen der vorhergehenden Ansprüche, **dadurch gekennzeichnet, dass** der Behälter (2) eine Öffnung (23) aufweist, die es ermöglicht, ihn mit einem chemischen Stoff zu füllen.

9. Vorrichtung zur Entnahme biologischen Gewebes nach einem beliebigen der vorhergehenden Ansprüche, **dadurch gekennzeichnet, dass** der Behälter (2) einen chemischen Stoff zur Behandlung einer Probe biologischen Gewebes enthält, vorzugsweise einen Konservierungsstoff, der dazu geeignet ist, eine Probe biologischen Gewebes zu konservieren.

10. Vorrichtung zur Entnahme biologischen Gewebes nach Anspruch 9, **dadurch gekennzeichnet, dass** der Konservierungsstoff aus der Gruppe gewählt ist, die Trocknungsmittel, Enzymhemmstoffe und Substanzen, welche die Entwicklung bakterieller Organismen verhindern können, umfasst.

11. Vorrichtung zur Entnahme biologischen Gewebes nach einem beliebigen der Ansprüche 9 oder 10, **dadurch gekennzeichnet, dass** die Öffnung (23) des Behälters (2) durch eine aufgesiegelte Metallfolie (24) verschlossen ist.

12. Vorrichtung zur Entnahme biologischen Gewebes nach einem beliebigen der vorhergehenden Ansprüche, **dadurch gekennzeichnet, dass** der Behälter (2) an seinem äußeren Abschnitt ein Rundstück (25) umfasst, das es ermöglicht, die Vorrichtung zur Entnahme am Steckelement einer Ohrmarke zur Kennzeichnung von Tieren zu verwenden, wobei das Rundstück Abmessungen hat, die es ermöglichen, dass dieses in eine der Wirkstellen einer Zange, die zum Aufziehen derartiger Marken verwendet wird, eingeführt wird.

13. Vorrichtung zur Entnahme biologischen Gewebes nach einem beliebigen der vorhergehenden Ansprüche, **dadurch gekennzeichnet, dass** die Vorrichtung zur Entnahme biologischen Gewebes ein erstes Mittel zur Kennzeichnung (5) aufweist.

14. Vorrichtung zur Entnahme biologischen Gewebes nach einem beliebigen der vorhergehenden Ansprüche, **dadurch gekennzeichnet, dass** die Wand (21) des Behälters (2) Mittel (213) zur Verbindung mit einem Deckel (6) in Form einer Kappe aufweist.

15. Vorrichtung zur Entnahme biologischen Gewebes nach Anspruch 14, **dadurch gekennzeichnet, dass** die Mittel zur Verbindung (213) auf der Außenseite an der Basis (215) des Behälters (2) angeordnet sind.

16. Vorrichtung zur Entnahme biologischen Gewebes nach einem beliebigen der Ansprüche 14 oder 15, **dadurch gekennzeichnet, dass** die Basis (215) des Behälters (2), welche die Mittel zur Verbindung (213) mit dem Deckel (6) in Form einer Kappe aufweist, einstückig durch das Teil zur Befestigung (22) der Hohlnadel (3) verlängert wird.

17. Vorrichtung zur Entnahme biologischen Gewebes nach einem beliebigen der Ansprüche 14 bis 16, **dadurch gekennzeichnet, dass** die Mittel zur Verbindung (213) eine Flansch (214) aufweisen, der dazu geeignet ist, den Deckel in Form einer Kappe derart festzuhalten, dass dieser den distalen Abschnitt (38) der Hohlnadel (3), die möglicherweise eine Probe biologischen Gewebes enthält, bedeckt.

18. Vorrichtung zur Entnahme biologischen Gewebes nach einem beliebigen der Ansprüche 14 bis 17, **dadurch gekennzeichnet dass** sie weiterhin einen Deckel (8) in Form einer Kappe umfasst, der auf den Flansch (214) der Vorrichtung zur Entnahme biologischen Gewebes montiert ist.

19. Vorrichtung zur Entnahme biologischen Gewebes nach Anspruch 18, **dadurch gekennzeichnet, dass** der Deckel (6) in Form einer Kappe eine Stützvorrichtung (51) für das erste Mittel zur Kennzeichnung (5) aufweist, wobei das erste Mittel zur Kennzeichnung sich ausgehend von der Vorrichtung zur Entnahme als Verlängerung erstreckt.

20. Vorrichtung zur Entnahme biologischen Gewebes nach einem beliebigen der Ansprüche 18 oder 19, **dadurch gekennzeichnet, dass** der Deckel (6) in Form einer Kappe aus einem durchsichtigen Material hergestellt ist, das es ermöglicht, die biologische Probe zu betrachten.

21. Ohrmarke zur Kennzeichnung von Tieren, aufweisend i) ein Steckbuchsenelement und ii) ein Steckelement (7), das einen Schaft (9) umfasst, der mit einer distalen Spitze (91) versehen ist, welche einen Ansatz (911) aufweist, der es ermöglicht, das Steckelement in dem Steckbuchsenelement einzurasten, wobei der Schaft auf seiner gesamten Länge von einem axialen Kanal (92) durchstochen ist, und wobei das Steckelement eine Vorrichtung zur Entnahme biologischen Gewebes (1) nach einem beliebigen der Ansprüche 1 bis 20 umfasst.

22. Ohrmarke nach dem vorhergehenden Anspruch, **dadurch gekennzeichnet, dass** die Hohlnadel (3) der Vorrichtung zur Entnahme biologischen Gewebes sich in dem axialen Kanal (92) des Schafts (9) befindet, wobei die Länge desselben derart beschaffen ist, dass die Schnittkante (36) des zweiten Endes (32) der Hohlnadel über die Spitze (91) des Schafts hinausreicht, sodass eine Probe biologischen Gewebes abgeschnitten wird, wenn das Steckelement (7) durch das Ohr eines Tieres gedrückt wird.

23. Ohrmarke nach einem der Ansprüche 21 oder 22, **dadurch gekennzeichnet, dass** das zweite Ende (32) der Hohlnadel (3) die Spitze (91) des Schafts (22) um eine Länge übertrifft, die mindestens gleich der Dicke der Trennwand (211) ist.

24. Ohrmarke nach einem der Ansprüche 21 bis 23 **dadurch gekennzeichnet, dass** sie weiterhin mindestens eines der folgenden Elemente umfasst:
- ein zweites Mittel zur Kennzeichnung (81), das sich auf einer Platte (8) befindet, die mit dem Schaft (9) verbunden ist,
- ein drittes Mittel zur Kennzeichnung (111), das sich auf dem Steckbuchsenelement befindet,
weshalb mindestens eines der zweiten oder dritten Mittel zur Kennzeichnung in der Ohrmarke vorhanden ist.
